# EUROPÄISCHE PATENTSCHRIFT

(11) **EP 2 921 110 B1**
(45) Veröffentlichungstag und Bekanntmachung des Hinweises auf die Patenterteilung: **07.10.2020**
(21) Anmeldenummer: 15159337.3
(22) Anmeldetag: 17.03.2015
(51) Int. Cl.: A61B 5/06, A61B 1/00, A61B 90/00, G01D 5/347

(54) **AUTOMATISCHE ERFASSUNG DER EINDRINGTIEFE UND DER ROTATORISCHEN ORIENTIERUNG EINES INVASIVEN INSTRUMENTS**
AUTOMATIC CAPTURE OF THE PENETRATION DEPTH AND THE ROTATIONAL ORIENTATION OF AN INVASIVE INSTRUMENT
DÉTECTION AUTOMATIQUE DE LA PROFONDEUR DE PÉNÉTRATION ET DE L'ORIENTATION ROTATIVE D'UN INSTRUMENT INVASIF

(30) Priorität: 19.03.2014 DE 102014103728
(43) Veröffentlichungstag der Anmeldung: 23.09.2015
(73) Patentinhaber: Karl Storz SE & Co. KG, 78532 Tuttlingen (DE)
(72) Erfinder: Gruhler, Ulrich, 78532 Tuttlingen (DE)

(56) Entgegenhaltungen:
- WO-A1-2013/115231
- US-A1- 2003 069 474
- US-A1- 2005 075 558
- US-A1- 2005 272 971
- US-A1- 2007 249 901
- US-A1- 2008 262 473

## Beschreibung

Die Erfindung betrifft eine Vorrichtung zur automatischen Erfassung der Eindringtiefe eines invasiven Instrumentes in eine Öffnung und der rotatorischen Orientierung eines invasiven Instrumentes in einer Öffnung eines Körpers sowie ein System zur automatischen Erfassung der Eindringtiefe und der rotatorischen Orientierung eines invasiven Instrumentes.

Aus der DE 34 24 806 C2 ist ein automatisiertes Digital-Nivelliergerät bekannt, das eine elektronische Lattenablesung ermöglicht. Zu diesem Zweck wird auf der Nivellierlatte ein Codemuster aus schwarzen und weißen Elementen aufgebracht, von dem ein Teil mithilfe der Fernrohroptik des elektronischen Nivelliergerätes auf einem ortsauflösenden Detektor abgebildet wird. Hierbei wird die im Gesichtsfeld des Fernrohrs befindliche Information des Codemusters genutzt, um durch Vergleich mit dem als Referenzcodemuster im Nivelliergerät abgespeicherten Codemuster der Nivellierlatte den gewünschten Höhenmesswert zu erhalten.

Weiterhin ist aus der Patentschrift DE 198 12 609 C2 ein Verfahren zur Bestimmung der Position und der Drehlage eines Operationsmikroskops oder eines Operationswerkzeuges, wie z.B. ein Skalpell oder ein Endoskop bekannt. Hierzu wird das zu untersuchende Objekt mithilfe von mehreren im Raum verteilten Kameras erfasst und aus den Bildinformationen die räumlichen Koordinaten einschließlich der Drehlage des zu untersuchenden Objektes ermittelt. Mithilfe von Informationen des Körpers des Patienten hinsichtlich dessen Position und Lage lässt sich die genaue räumliche Lage des zu untersuchenden Objektes, insbesondere des Operationsmikroskops oder der Operatiorninstrumente bezüglich des Operationsgebietes des Menschen erfassen, auswerten und anzeigen. Mithilfe dieser Information ist es auch möglich, die Operationsinstrumente vollautomatisch zu führen. Dieses Verfahren erweist sich als sehr komplex und aufwändig, da es eine umfangreiche Menge an Daten zu dem zu operierenden Körper und zu den durch mehrere kameraartige Messköpfe erfassten Bildinformationen verarbeiten muss, um eine verlässliche Information zur Eindringtiefe und zur rotatorischen Orientierung eines medizinischen invasiven Instrumentes oder Objektes zu bestimmen.

Darüber hinaus sind flexible Endoskope und flexible Boreskope bekannt, die auf ihrem flexiblen Schaft Strichmarkierungen mit Längenangaben zeigen, die den Abstand der Strichmarkierung zu dem distalen Ende des Schaftes angeben. Mithilfe dieser Markierung kann der Benutzer des flexiblen Endoskops bei einer Operation, z.B. eines Menschen oder bei einem Boreskop die Eindringtiefe in den Körper oder z.B. in das Abwasserrohr abschätzen, indem er die letzte erkennbare Strichmarkierung mit Abstandsangabe im Bereich der Körperöffnung oder der Rohröffnung abliest. Diese Art der Abschätzung der Eindringtiefe ist sehr ungenau und nicht automatisiert.

Aus der US2005/0075558A1 ist eine Vorrichtung zur Erfassung einer translatorischen und einer rotatorischen Bewegung eines Instrumentenschafts bekannt. Ein optischer Sensor erfasst hierzu ein in Längsrichtung und umfänglich auf dem Schaft angebrachtes Muster.
Eine ähnliche Vorrichtung ist aus der WO2013/115231A1 bekannt. Aus dem Vergleich zweier aufgenommener Bilder wird Grad der Einführung in Längsrichtung bzw. ein Grad der Rotation des Schafts berechnet.
Die US2005/0272971A1 beschreibt ebenfalls eine solche Vorrichtung, wobei der Sensor außerdem in einen Bissschutz zur Einführung eines flexiblen Endoskops in den Patienten durch den Mund integriert sein kann.

Der Erfindung liegt die Aufgabe zugrunde, eine Vorrichtung zur automatischen Erfassung der Eindringtiefe und der rotatorischen Orientierung eines invasiven Instrumentes anzugeben, die eine verlässliche Information zur Eindringtiefe und zur rotatorischen Orientierung ermöglicht und dabei einen relativ einfachen Aufbau zeigt. Eine weitere Aufgabe der Erfindung liegt darin, ein System zur automatischen Erfassung der Eindringtiefe und der rotatorischen Orientierung eines invasiven Instrumentes mit den zuvor genannten Vorteilen anzugeben.

Diese erfindungsgemäßen Aufgaben werden durch eine Vorrichtung zur Erfassung der Eindringtiefe und der rotatorischen Orientierung eines invasiven Instrumentes mit den Merkmalen des Anspruchs 1 bzw. durch ein System zur automatischen Erfassung der Eindringtiefe und der rotatorischen Orientierung eines invasiven Instrumentes mit den Merkmalen des Anspruchs 12 gelöst. Vorteilhafte Weiterbildungen sind Gegenstand der abhängigen Ansprüche.

Die erfindungsgemäße Vorrichtung zur automatischen Erfassung der Eindringtiefe eines invasiven Instrumentes in eine Öffnung und der rotatorischen Orientierung eines invasiven Instrumentes in einer Öffnung eines Körpers ermöglicht es, mithilfe eines auf der Oberfläche des Instrumentes angeordneten längenselektiven und rotationsselektiven Musters die Eindringtiefe oder die rotatorische Anordnung zu erfassen und bei Bedarf einem Benutzer auszugeben. Dabei kann das invasive Instrument einerseits ein technisches Instrument sein, beispielsweise ein starres oder flexibles Boreskop, mit dem beispielsweise Untersuchungen eines Abwasserkanals oder eine Untersuchung beispielsweise einer Gasturbine erfolgen können, aber auch medizinische invasive Instrumente sein, beispielsweise flexible oder starre Endoskope, mittels derer minimalinvasive Operationen im menschlichen oder tierischen Körper durchgeführt werden können, oder auch endoskopische Instrumente, mittels derer Manipulationen im Rahmen minimalinvasiver Operationen vorgenommen werden können. Diese Manipulationen können beispielsweise ein einfaches Verschieben von Gewebe, ein Abtrennen und Entnehmen von Gewebe oder ein HF-Koagulieren beinhalten. Bei all diesen invasiven Instrumenten kann es für den Benutzer sehr wichtig sein, eine verlässliche Information über die Eindringtiefe des Instrumentes in die jeweilige Öffnung zu erhalten, sei es bei einer minimalinvasiven Operation eine Körperöffnung eines Menschen oder Tieres oder sei es eine technische Öffnung, z.B. eine Rohröffnung.

Die erfindungsgemäße Vorrichtung enthält einen Sensor, der ringförmig ausgebildet ist und der geeignet ist, das invasive Instrument, dessen Eindringtiefe und rotatorische Orientierung zu messen ist, zu umschließen. Der ringförmig ausgebildete Sensor erfasst insbesondere auf optischer Basis das längenselektive und rotationsselektive Muster auf dem invasiven Instrument und führt diese Information an eine Auswerteeinheit zur Auswertung der Eindringtiefe und der rotatorischen Orientierung des invasiven Instrumentes weiter.

Durch die erfindungsgemäße Anordnung im Bereich der Öffnung, insbesondere unmittelbar an der Öffnung des Körpers, durch die das invasive Instrument eingebracht wird, gelingt es, das dort befindliche Muster auf dem eingebrachten invasiven Instrument selektiv zu erfassen und die verlässliche Eindringtiefe bzw. die rotatorische Orientierung des Instrumentes zu erfassen und auszuwerten und für eine mögliche Ausgabe an den Benutzer zur Verfügung zu stellen. Die zeitweilige Anordnung des Sensors erfolgt dabei erfindungsgemäß so, dass der ringförmig ausgebildete Sensor mit der Öffnung des Körpers so in Überlapp gebracht wird, dass durch die überlappende Öffnung das invasive Instrument, dessen Eindringtiefe bzw. rotatorische Orientierung erfasst werden soll, eingeführt werden kann und eine aussagekräftige Information zur Eindringtiefe bzw. zur rotatorischen Orientierung des Instrumentes gemessen werden kann.

Durch das unmittelbare Messen, d.h. Erfassen und Auswerten des längenselektiven und rotationsselektiven Musters im Bereich der Öffnung des Körpers ist eine sehr aussagekräftige und einfache erfindungsgemäße Vorrichtung geschaffen, die unmittelbar und automatisch sowie verlässlich die notwendige Information wie Eindringtiefe und der rotatorischen Orientierung zum in die Öffnung des Körpers eingebrachten invasiven Instrument erfasst.

Erfindungsgemäß ist das Muster auf der Oberfläche des invasiven Instrumentes längenselektiv ausgebildet, d.h. das Muster verändert sich kontinuierlich oder quasi kontinuierlich mit dem Abstand zum distalen Ende des invasiven Instrumentes. Dabei ist das Muster in einem bestimmten Abstand für diesen Abstand selektiv und damit so eindeutig, dass anhand der Mustererfassung mithilfe des Sensors und der zugeordneten Auswerteeinheit eine Aussage über den Abstand zum distalen Ende der Position des Musterelementes gewonnen werden kann. Damit kann beispielsweise eine Information gewonnen werden, dass ein flexibles technisches Boreskop 7, 48 Meter in einen Kabelkanal eingeführt worden ist.

Mithilfe eines rotationsselektiven Musters auf der Oberfläche des Instrumentes, das über die ganze Länge oder zumindest einen Teil der Länge des invasiven Instrumentes angebracht ist und beispielsweise eine charakteristische Markierung an einer Position des Umfangs zeigt, gelingt es, diese charakteristische Markierung auf dem Umfang selektiv mithilfe des ringförmigen Sensors so zu erfassen, dass daraus mithilfe der Auswerteeinheit eine Information über die rotatorische Orientierung des invasiven Instrumentes gewonnen werden kann. Da diese charakteristische Markierung in dem ringförmigen Sensor durch diesen erfasst wird und dieser sich in unmittelbarer Nähe zur Öffnung befindet, kann damit eine Information über die rotatorische Orientierung des invasiven Instrumentes in der Öffnung des Körpers werden und anschließend eine Ausgabe oder eine anderswertige Nutzung dieser Informationen erfolgen.

Dabei hat es sich besonders bewährt, ein längen- und rotationsselektives Muster zu verwenden und diese beiden Informationen gemeinsam zeitlich auszuwerten, so dass durch die erfindungsgemäße Vorrichtung sowohl eine Information über die Eindringtiefe des invasiven Instrumentes in die Öffnung aber zugleich auch eine Information über die rotatorische Orientierung des invasiven Instrumentes in der Öffnung eines Körpers gewonnen werden kann und erfindungsgemäß einer Nutzung zugeführt werden kann. Diese Nutzung kann einerseits durch die entsprechende Information an den Benutzer, insbesondere den Operateur, beispielsweise mithilfe einer Anzeigeneinheit mittels optischer Anzeige, also in Form eines Displays erfolgen aber auch alternativ oder zusätzlich beispielsweise als Information zur Führung des invasiven Instrumentes erfolgen, was ggf. voll- oder teilautomatisiert erfolgen kann.

Eine andere Ausbildung der Erfindung zeigt ein System zur automatischen Erfassung der Eindringtiefe und der rotatorischen Orientierung eines invasiven Instrumentes mit einer erfindungsgemäßen Vorrichtung und einem invasiven Instrument, das auf seiner Oberfläche ein längenselektives und rotationsselektives Muster aufweist. Dieses Muster, das über die ganze oder einen Teil der Länge des invasiven Instrumentes aufgebracht ist, weist auf dem Umfang des Instrumentes Bereiche unterschiedlicher Farbe und/oder unterschiedlicher Helligkeiten auf. Dabei können die unterschiedlichen Bereiche des auf dem Umfang des Instrumentes angeordneten Musters zwei oder mehr unterschiedliche Farben und/oder unterschiedliche Helligkeiten aufweisen, die eindeutig voneinander unterscheidbar sind. Durch die erhöhte Anzahl von unterschiedlich verwendeten Farben oder Helligkeitsstufen, die auch kombiniert sein können, lässt sich der Informationsgehalt des Musters bei gegebener Anzahl von verwendeten Bereichen erheblich steigern. Werden beispielsweise fünf Bereiche verwendet und nur zwei unterschiedliche Farben verwendet, die mithilfe des Sensors erfasst werden können, so können maximal 2⁵, also 32 unterschiedliche selektive Informationen hinsichtlich der Länge bzw. der Orientierung mithilfe des Musters dargestellt werden. Werden jedoch 10 unterschiedliche Farben und/oder Helligkeitsstufen verwendet, so sind es bei fünf verwendeten Bereichen bereits 10⁵, also 100.000 unterschiedliche Informationen zur Länge bzw. Orientierung. Das Muster wird dabei entlang des invasiven Instrumentes so angebracht, dass die auf dem Umfang angebrachte Anordnung der Bereiche unterschiedlicher Farbe oder Helligkeit selektiv abhängig von dem Abstand zum distalen Ende des invasiven Instrumentes ist und damit eine eindeutige Abstandsinformation repräsentiert. Bezüglich der Information zur rotatorischen Orientierung wird auf das zuvor beschriebene Konzept der charakteristischen Markierung einer Umfangsposition verwiesen. Zeigt das längen selektive Muster zusätzlich eine charakteristische Position auf dem Umfang, ist es möglich, nicht nur die längenselektive Information zu erfassen und auszuwerten, sondern auch zusätzlich die rotatorische Orientierung zu erfassen und auszuwerten. Ein Aufaddieren der Längeninformationen vom einem Schaftende her, um die aktuelle Position zu ermitteln, ist dabei nicht notwendig.

Durch das erfindungsgemäße Zusammenwirken des invasiven Instrumentes mit dem charakteristischen, über seine Länge oder einen Teil seiner Länge angeordneten, längenselektiven und rotationsselektiven Muster mit der Vorrichtung zur Erfassung der Eindringtiefe und der rotatorischen Orientierung des invasiven Instrumentes gelingt es, eine sehr verlässliche und sichere sowie einfache Information zur Eindringtiefe oder zur rotatorischen Orientierung zu gewinnen und diese einer weiteren Verwendung zuzuführen. Beispielsweise können Eindringtiefe und rotatorische Position zusammen mit einem Zeitstempel oder einem an dieser Position aufgenommenen Bild gespeichert werden. So kann später ein Bild einer bestimmten Position des Instruments im Körper zugeordnet werden.

Ein bevorzugtes System zur automatischen Erfassung der Eindringtiefe und der rotatorischen Orientierung eines invasiven Instrumentes verwendet auf der Oberfläche des invasiven Instrumentes ein binäres Muster aus mehreren Bereichen von zwei unterschiedlichen Farben und/oder Helligkeiten, z.B. schwarz und weiß, wobei die verschiedenen auf dem Umfang des Instrumentes angebrachten Anordnungen aus den mehreren Bereichen so gewählt sind, dass sie eine Hamming-Distanz von wenigstens 1 aufweisen. Dadurch gelingt es, dass sich die Anordnungen an verschiedenen Längspositionen so deutlich unterscheiden, dass selbst bei einem Auslesefehler die enthaltene Information zur Eindringtiefe respektive der Abstand zum distalen Ende des invasiven Instrumentes dennoch eindeutig und verlässlich erfasst und ausgewertet und einer weiteren Verwendung zugeführt werden kann. Dies führt dazu, dass erfindungsgemäß auf einen Teil der möglichen Anordnungen verzichtet wird und dadurch die Auflösung der Längeninformation bzw. der Orientierungsinformation merklich eingeschränkt ist. Beispielsweise können bei dieser erfindungsgemäßen Anordnung der Bereiche des Musters in Form eines Binärcodes, beispielsweise mit fünf Bits, also fünf Bereichen nicht 2⁵, also 32 Auflösungsschritte verwendet werden, sondern ausschließlich sechs verschiedene Anordnungen mit der besagten Hamming-Distanz verwendet werden. Hierdurch wird im Hinblick auf die erhöhte Verlässlichkeit und Aussagekraft der Information zur Eindringtiefe und der rotatorischen Orientierung ein Verzicht auf die Auflösung erfindungsgemäß in Kauf genommen. Dies hat sich gerade bei der Verwendung von starren invasiven Instrumenten, wie z.B. bei starren Endoskopen als durchaus ausreichend erwiesen. Die Verwendung von binären Codes hat sich als sehr sicher erwiesen, da die Sensoren die unterschiedlichen Bereiche sehr sicher unterscheiden können. Die dadurch bedingte geringere Auflösung, insbesondere hinsichtlich der gewünschten Hamming-Distanz der verwendeten Musteranordnungen wird dadurch kompensiert, dass die Anzahl der verwendeten Bereiche der Muster erhöht wird. Gerade bei kurzen starren, invasiven Instrumenten werden wenigstens fünf Bereiche für ein binäres Muster mit 5 Bit Länge verwendet, bei längeren invasiven Instrumenten werden 10 oder mehr Bereiche zur Erhöhung der Auflösung verwendet.

Darüber hinaus hat es sich besonders bewährt, das System zur automatischen Erfassung der Eindringtiefe und rotatorischen Orientierung eines invasiven Instrumentes so weiterzubilden, dass das verwendete Muster auf dem Umfang des invasiven Instrumentes wenigstens einen Übergang von einer zur anderen Farbe und/oder Helligkeit und wenigstens einen umgekehrten Übergang aufweist. Als besonders bevorzugt hat es sich erwiesen, nur solche Anordnungen der Bereiche im Muster zu verwenden, die auch bei zyklischem Vertauschen der Bereiche eindeutig sind. Dadurch gelingt es, zusätzlich zu den Informationen zur Eindringtiefe auch die rotatorische Orientierung darzustellen, da jede Anordnung auf dem Umfang des Instruments für sich eine eindeutige Reihenfolge der Bereiche hat, die sich auch bei zyklischem Vertauschen der Bereiche von allen anderen Anordnungen unterscheidet. Dadurch ist sichergestellt, dass bei einem über den vollen Umfang des invasiven Instrumentes angebrachten durchgängigen Muster unabhängig von dem Beginn der Auswertung entlang des Umfangs jeweils eine eindeutige und selektive Anordnung gegeben ist, und eine eindeutige Information zur rotatorischen Orientierung des Instruments erreicht und gewonnen werden kann.

Eine vorteilhafte Weiterbildung der erfindungsgemäßen Vorrichtung zeichnet sich dadurch aus, dass sie ein ringförmiges Gehäuse aufweist, in dem der ringförmige Sensor angeordnet ist und zusätzlich zu dem ringförmigen Sensor eine Auswerteeinheit im Gehäuse integriert ist. Der ringförmige Sensor, der das invasive Instrument mit dem längen- bzw. rotationsselektiven Muster auf dessen Oberfläche umschließt und dieses Muster erfasst, gibt sein Sensorsignal an die Auswerteeinheit in dem gemeinsamen ringförmigen Gehäuse weiter, so dass die Auswertung der Sensorsignale als Eindringtiefeninformation oder rotatorische Orientierungsinformation innerhalb des kompakten ringförmigen Gehäuses durchgeführt wird, das typischerweise eine erfindungsgemäße Vorrichtung bildet. Durch diese Ausbildung der erfindungsgemäßen Vorrichtung ist eine sehr kompakte Einheit geschaffen, die ein Anbringen im Bereich der Öffnung gerade bei Operationen an einem Menschen oder einem Tier ermöglicht, ohne dass eine relevante Behinderung der Operateure zu befürchten ist. Zudem erweist sich diese erfindungsgemäße Vorrichtung durch diese ringförmige Ausbildung des Gehäuses als gut sterilisierbar und damit für den medizinischen Einsatz geeignet. Weiterhin gewährleistet das ringförmige Gehäuse einen verlässlichen und sicheren Schutz des im Gehäuse integrierten ringförmigen Sensors mit Auswerteeinheit.

Es hat sich als besonders vorteilhaft erwiesen, die erfindungsgemäße Vorrichtung so auszubilden, dass das ringförmige Gehäuse aufklappbar ausgebildet ist. Dabei kann das ringförmige Gehäuse mit dem integrierten ringförmigen Sensor aus mehreren Ringteilen zusammengesetzt sein, die voneinander getrennt werden können, wobei diese teilweise durch flexible, insbesondere scharnierförmige Verbindungselemente miteinander verbunden bleiben können und damit um diese Verbindungsstelle gegeneinander verschwenkt werden können. Durch diese mehrteilige Ausbildung des Gehäuses zum Aufklappen wird es möglich, die Vorrichtung auf einfache und verlässliche Weise an dem Einsatzort, d.h. im Bereich der Öffnung um das invasive Instrument, das ggf. bereits in die Öffnung des Körpers eingebracht wurde, so zusammenzusetzen und so anzuordnen, dass das ringförmige Gehäuse mit dem ringförmigen Sensor das zu erfassende invasive Instrument ringförmig umschließt und dadurch die Oberfläche über den ganzen Umfang des invasiven Instrumentes mittels des ringförmigen umschließenden Sensors erfassen und ausgewertet werden kann. Dadurch ist eine sehr sichere und einfache Handhabung auch unter schwierigen Bedingungen gewährleistet. Insbesondere ermöglicht diese erfindungsgemäße Vorrichtung auch eine nachträgliche Anbringung der erfindungsgemäßen Vorrichtung in einem Fall, bei dem erst in einem späteren Stadium der Operation der Bedarf einer Eindringtiefeninformation oder Orientierungsinformation notwendig geworden ist. In diesem Fall ist eine Entnahme des invasiven Instrumentes aus der Öffnung entbehrlich, was die Handhabung wesentlich erleichtert und eine Beschädigung des Instrumentes bzw. des Körpers weniger wahrscheinlich macht. Zudem gelingt es, die Handhabungszeit des invasiven Instrumentes deutlich zu reduzieren.

Durch das fakultative Vorsehen eines optisch transparenten Innengehäuses auf der Innenseite der Vorrichtung bzw. des ringförmigen Gehäuses der Vorrichtung, gelingt es einerseits, eine optische Erfassung des Musters auf der Oberfläche eines in das ringförmige Gehäuse bzw. in die ringförmige Vorrichtung eingebrachten invasiven Instrumentes verlässlich zu gewährleisten und andererseits durch das transparente Innengehäuse eine mechanische verlässliche Trennung des empfindlichen ringförmigen Sensors von dem invasiven Instrument bzw. der Umgebung, insbesondere der Öffnung, zu gewährleisten. Dadurch kann einerseits eine Beschädigung des Sensors ausgeschlossen werden, aber auch im Falle einer medizinischen Anwendung der Vorrichtung kann eine Beeinträchtigung des Operationsumfeldes z.B. durch einen Einfluss der Substanzen, insbesondere der metallischen Substanzen oder chemischen Substanzen des integrierten ringförmigen Sensors oder der Auswerteeinheit, die durch das Gehäuse bzw. das transparente Innengehäuse abgetrennt sind, verhindert werden. Eine Kontamination des zu operierenden Körpers kann damit weitgehend ausgeschlossen werden. Im Übrigen erweist sich durch die geeignete Wahl des Materials des transparenten Innengehäuses auch die Vorrichtung als besonders gut zu reinigen und insbesondere zu sterilisieren, was den Einsatzbereich der Erfindung und die Qualität der erfassten Eindringtiefeninformation bzw. rotatorischen Orientierungsinformation wesentlich erhöht.

Neben der Möglichkeit, einen einzigen einstückigen länglichen Sensor ringförmig in der Vorrichtung anzuordnen, hat es sich als vorteilhaft erwiesen, mehrere einzelne Sensorelemente zu verwenden, die gemeinsam den Sensor bilden, und diese so ringförmig in der Vorrichtung anzuordnen, dass sie den Innenbereich des Rings vollständig in einer Weise erfassen können, dass ein in diesen Innenbereich eingebrachtes invasives Instrument mittels des aus mehreren Sensorelementen gebildeten ringförmigen Sensors sensiert werden kann. Damit ist sichergestellt, dass ein auf der Oberfläche des invasiven Instrumentes angebrachtes selektives Muster verlässlich und sicher erfasst und einer Auswertung zugeführt werden kann. Hierdurch können sehr kompakte optimierte Einzelsensorelemente zu einem ringförmigen Gesamtsensor verbunden werden, indem beispielsweise die Sensorelemente über eine gemeinsame Verschaltung elektrisch miteinander verbunden sind. Die Verschaltung kann beispielsweise durch eine ringförmige Leiterplatte, die insbesondere als ringförmige flexible Leiterplatte ausgebildet ist, erfolgen. Die Signale der einzelnen Sensorelemente werden durch die Leiterplatte der insbesondere auf der Leiterplatte angeordneten Auswerteeinheit zugeführt und dort gemeinsam zu dem Sensorsignal zusammengefasst und zu einer Information über die Eindringtiefe und die rotatorische Orientierung ausgewertet.

Dabei hat es sich als besonders vorteilhaft erwiesen, die einzelnen Sensorelemente als einzelne CCD-Sensorelemente, CMOS- Sensorelemente und/oder Reflexlichtschranken-Sensorelemente zu auszubilden. Diese Sensorelemente erweisen sich für diese Anwendung als sehr geeignet, da sie einerseits sehr kompakt ausgebildet werden können und besonders geeignet sind, ein erfindungsgemäß selektives Muster auf der Oberfläche eines invasiven Instrumentes im Nahbereich, also im Innenbereich des ringförmigen Sensors verlässlich zu erfassen. Neben der Möglichkeit, ausschließlich eine einzige Art von Sensorelementen zu verwenden, hat es sich auch bewährt, Kombinationen unterschiedlicher Arten an beispielhaften Sensorelementen gemeinsam zu verwenden. Bevorzugt werden dabei die Sensorelemente so angeordnet und ausgebildet bzw. ausgewählt, dass sie wechselseitig einen Überlapp ihrer Erfassungsbereiche aufweisen, so dass auch beim Ausfall eines einzelnen Sensorelements eine verlässliche Erfassung des Musters gewährleistet ist. Die zusätzliche ggf. mehrfache Information der verschiedenen Sensorelemente wird dabei mithilfe der Auswerteeinheit so ausgewertet, dass diese mehrfache Information nicht zu einer Fehlinformation führt.

Weiterhin hat es sich bewährt, den ringförmigen Sensor zu realisieren, indem mehrere Lichtwellenleiter so verwendet werden, dass ihre einen Enden im Gehäuse ringförmig so angeordnet sind, dass ihre Enden in Richtung Innenraum der ringförmigen Anordnung der Enden der Lichtwellenleiter zeigen und Licht vom Innenraum aufnehmen können. Die anderen Enden der Lichtwellenleiter sind jeweils einem oder mehreren Sensorelementen so zugeordnet, dass die Sensorelemente das vom Innenraum durch die Lichtwellenleiter übertragene Licht erfassen und sensieren können. Dabei sind die Sensorelemente arrayförmig angeordnet, wobei dies bevorzugt als lineares Array erfolgt. Dadurch lässt sich eine sehr kompakte Anordnung aus Sensorelementen erreichen, die einerseits gut in ein gemeinsames Gehäuse, insbesondere in das ringförmige Gehäuse integrierbar ist.

Das Array aus Sensorelementen wird bevorzugt auf einer Leiterplatte angeordnet, auf der auch die Auswerteeinheit angebracht ist. Dadurch lässt sich die Anzahl der Komponenten reduzieren und eine verlässliche und sichere Integration in die Vorrichtung mit Gehäuse gewährleisten. Als besonders bevorzugte Sensorelemente haben sich CCD-Sensorelemente, CMOS-Sensorelemente und/oder Reflexlichtschranken-Sensorelemente bewährt. Neben der ausschließlichen Verwendung eines einzelnen Typs von Sensorelementen hat sich auch eine Kombination unterschiedlicher Varianten eines Typs von Sensorelementen, beispielsweise unterschiedliche CCD-Sensorelemente aber auch eine Kombination unterschiedliche Technologiearten von Sensorelementen wie CCD-Sensorelemente mit Reflexlichtschranken-Sensorelementen bewährt. Hierdurch lassen sich die Einsatzbereiche der Erfindung durch die passende Wahl der verwendeten Sensorelemente erweitern.

In einer weiteren Ausgestaltung weist das ringförmige Gehäuse mit dem ringförmigen Sensor eine insbesondere ringförmige Lichtquelle auf, die den durch das ringförmige Gehäuse umschlossenen Innenbereich ringförmig beleuchtet. Die Beleuchtung erfolgt insbesondere durch das transparente Innengehäuse.

Durch das Vorsehen einer bevorzugt ringförmig ausgebildeten Lichtquelle zur Beleuchtung des Innenraums des ringförmigen Sensors der erfindungsgemäßen Vorrichtung gelingt es, den Einsatzbereich der erfindungsgemäßen Vorrichtung auch unter schwierigen äußeren Umständen, also bei geringem oder nicht vorhandenem äußerem Licht zu gewährleisten. Dadurch gelingt es, unabhängig von äußeren Lichtquellen zu sein und dadurch die Voraussetzung für ein sicheres Erfassen und Erkennen der selektiven Muster auf einem eingebrachten invasiven Instrument zu gewährleisten. Durch die eigene Lichtquelle, die insbesondere als ringförmige Lichtquelle realisiert ist, ist erfindungsgemäß unabhängig von äußeren weiteren Gegebenheiten eine ausreichende Beleuchtung der Oberfläche mit dem selektiven Muster des eingebrachten invasiven Instrumentes gewährleistet. Gerade die Ausbildung einer ringförmigen Lichtquelle, die in ihrer Anordnung im Gehäuse dem ringförmigen Sensor entspricht, gewährleistet einerseits eine sichere vollflächige Beleuchtung des Innenraums der ringförmigen Anordnung und zugleich eine entsprechende vollflächige Erfassung des Innenraums der ringförmigen Anordnung des Sensors. Dabei wird in diese einander entsprechenden Innenräume das zu erfassende invasive Instrument eingebracht. Besonders vorteilhaft hat sich dabei erwiesen, ein transparentes Innengehäuse vorzusehen, das die insbesondere ringförmige Lichtquelle mit dem ringförmigen Sensor von dem Innenraum verlässlich mechanisch, chemisch und biologisch trennt und dadurch wechselseitige ungewollte Einflussnahme verhindert.

Neben der Möglichkeit, eine einzige ringförmige Lichtquelle vorzusehen, hat es sich gerade bei dem Vorsehen eines aufklappbaren ringförmigen Gehäuses bewährt, mehrere Einzellichtquellen vorzusehen, die ringförmig angeordnet sind, gemeinsam die ringförmige Lichtquelle bilden und dadurch den Innenraum des ringförmigen Sensors möglichst vollständig beleuchten.

Die Energieversorgung und Ansteuerung der Einzellichtquellen erfolgt bevorzugt über dieselbe Struktur, z.B. in Form einer ringförmigen Leiterplatte, wie bei dem ringförmigen Sensor bzw. den ringförmig angeordneten Sensorelementen. Dadurch lassen sich eine hohe Integration und eine Reduktion der Komponenten erreichen, was das Volumen der Vorrichtung reduziert und ein kompaktes ringförmiges Gehäuse für diese Vorrichtung ermöglicht.

Daneben hat es sich auch bewährt, die Beleuchtung des Innenraums mithilfe von Lichtwellenleitern zu gewährleisten, in die Licht mittels einer oder mehrerer zentraler Lichtquellen an einem Ende eingekoppelt wird und über die Lichtwellenleiter jeweils zum anderen Ende geführt wird und dort ausgekoppelt wird. Diese anderen Enden sind dabei ringförmig um den Innenraum des ringförmigen Sensors angeordnet, wodurch eine möglichst vollständige Beleuchtung dieses Innenraums erfolgt. Bevorzugt werden Lichtwellenleiter nicht nur zur Beleuchtung verwendet, sondern parallel dazu andere Lichtwellenleiter zur Sensierung. Dabei können diese in ihrer Funktion unterschiedlichen Lichtwellenleiter alternierend zueinander ringförmig um den Innenraum angeordnet werden und dadurch eine sehr hohe Dichte an Lichtwellenleiterfaserenden erreicht werden. Dies führt zu einer sehr hohen Auflösung des ringförmigen Sensors mit gleichmäßiger und starker Beleuchtung. Erfindungsgemäß können mehrere Sensorlichtwellenleiter auf einen Beleuchtungslichtwellenleiter in einer gemeinsamen ringförmigen Anordnung kommen, das heißt, neben der Möglichkeit mehrere, voneinander getrennte ringförmige Anordnungen zu bilden wird bevorzugt eine einzige gemeinsame ringförmige Anordnung aus alternierenden Lichtwellenleiterenden gewählt.

Um die Verlässlichkeit der Information zur Eindringtiefe und zur rotatorischen Orientierung zu erhöhen, hat es sich besonders bewährt, nicht nur einen einzigen ringförmigen Sensor zur Erfassung des längenselektiven und rotationsselektiven Musters vorzusehen, sondern mehrere insbesondere zwei oder drei solcher ringförmiger Sensoren vorzusehen, die parallel zueinander und beabstandet zueinander angeordnet sind. Jeder dieser ringförmigen Sensoren ist geeignet, den Innenbereich seiner ringförmigen Sensorstruktur zu erfassen und einer Auswertung zuzuführen. Dadurch gelingt es, dass unterschiedliche, voneinander beabstandete Bereiche eines in den Innenbereich eingebrachten invasiven Instrumentes mit aufgebrachtem selektivem Muster parallel zueinander erfasst werden. Abhängig von den unterschiedlichen Erfassungsbereichen erfassen die verschiedenen Sensoren jeweils unterschiedliche selektive Muster des Gesamtmusters, was zu typisch unterschiedlichen Sensorinformationen führt. Beispielsweise ergeben sich unterschiedliche Eindringtiefen bzw. Abstände zum distalen Ende des invasiven Instrumentes, da die Erfassungsbereiche zueinander versetzt sind und sich entsprechend unterscheiden. Die Auswerteeinheit berücksichtigt bei deren Auswertung der verschiedenen Sensorsignale die Abstände der ringförmigen Sensoren zueinander, so dass eine Korrektur der sensierten Werte erfolgt und eine einheitliche, korrekte Information zur Eindringtiefe und zur rotatorischen Orientierung gewonnen werden kann. Diese erweist sich als besonders verlässlich und sicher.

Die elektrische und ggf. optische Verbindung der einzelnen Komponenten der erfindungsgemäßen Vorrichtung erfolgt bevorzugt mithilfe einer in dem Gehäuse angeordneten Leiterplatte, die insbesondere ringförmig oder zumindest teilringförmig ausgebildet ist und den ringförmigen Sensor bzw. die ringförmige Lichtquelle zumindest teilweise umschließt. Durch diese Art der ringförmigen oder teilringförmigen Leiterplatte gelingt es, eine sehr verlässliche und sichere Verbindung der elektrischen bzw. elektronischen oder optoelektronischen Komponenten der Vorrichtung zu gewährleisten, die in Verbindung mit dem bevorzugt ringförmigen Gehäuse auch eine zusätzliche mechanische Stabilität und Integrität gewährleistet. Durch die Verwendung dieser ringförmigen oder teilringförmigen Leiterplatte, die auch als flexible Leiterplatte insbesondere als teilflexible Leiterplatte ausgebildet sein kann, gelingt es, die Vorrichtung mit ihrem Gehäuse, das bevorzugt ringförmig ausgebildet ist, sehr kompakt zu gestalten und dadurch gerade im medizinischen Umfeld gut einsetzbar zu machen.

Neben der Möglichkeit, die Vorrichtung mit dem Gehäuse mit einer eigenen nicht abgesetzten Ausgabeeinheit zu versehen und die ausgewerteten Informationen zur Eindringtiefe und zur rotatorischen Orientierung am Gehäuse der Vorrichtung selbst auszugeben, hat es sich besonders bewährt, eine abgesetzte Ausgabeeinheit vorzusehen, die bevorzugt drahtlos verbunden ist. Diese Ausgabe ist geeignet, die ausgewerteten Informationen zur Eindringtiefe in einen Körper und zur rotatorischen Orientierung des invasiven Instrumentes im Körper einem Benutzer zur Verfügung zu stellen, der beispielsweise der Operateur bei einer Operation eines Menschen ist. Dies erfolgt bevorzugt mithilfe einer optischen Darstellung, beispielsweise mithilfe eines Displays. Bei dem Display wird die Eindringtiefe bevorzugt in einer alphanumerischen Darstellung angegeben, alternativ kann auch eine grafische, symbolische Darstellung verwendet werden. Demgegenüber wird bevorzugt die rotatorische Orientierung in Form einer grafischen, symbolischen Darstellung verwendet, wohingegen die alternative alphanumerische Darstellung, beispielsweise in Winkelgraden, möglich aber weniger gebräuchlich ist. Die Erfassung der exakten Eindringtiefe, insbesondere in Zentimetern oder Teilen von Zentimetern, ist dabei von größerer Bedeutung, weshalb sich die alphanumerische Darstellung besonders bewährt hat. Die symbolische Darstellung der rotatorischen Orientierung ist gegenüber der alphanumerischen Darstellung für den Benutzer leichter und schneller und dadurch verlässlicher erfassbar. Zusätzlich oder alternativ hat es sich auch bewährt, die auszugebenden Informationen auch akustisch dem Benutzer zur Verfügung zu stellen.

Die drahtlose Übertragung der Informationen von der Vorrichtung zu der Auswerteeinheit erweist sich als die bevorzugte Art der Übertragung, wobei sich auch eine drahtgebundene Übertragung als geeignet erwiesen hat, da eine Verwendung von drahtlosen Übertragungstechniken im Operationssaal immer ein gewisses elektromagnetisches Störpotenzial für andere lebenswichtige Komponenten des Operationssaals beinhaltet.

Bei dem Einsatz der erfindungsgemäßen Vorrichtung wird die Vorrichtung im Bereich der Öffnung des Körpers angeordnet, in die das invasive Instrument eingebracht wird. Dabei hat es sich gerade bei einer Verwendung im medizinischen Umfeld bewährt, das ringförmige Gehäuse der erfindungsgemäßen Vorrichtung für eine, insbesondere lösbare Befestigung an dem Körper im Bereich der Öffnung und hier insbesondere unmittelbar an der Öffnung des Körpers vorzusehen. Die lösbare Befestigung erfolgt dabei bevorzugt durch eine Klebeverbindung, beispielsweise in der Art eines Pflasters, oder durch eine bandförmige Fixierung, beispielsweise mittels einer Schlaufe oder mittels Unterdruckflächen, mittels derer sich das Gehäuse an dem Körper, insbesondere an der beispielsweise menschlichen Haut festsaugt und dadurch eine verlässliche, insbesondere lösbare Befestigung im Bereich der Öffnung gewährleistet. Durch die lösbare Realisierung der Befestigung wird es möglich, die Vorrichtung rückstandsfrei von dem Körper zu entfernen. Hierzu wird die Vorrichtung bzw. das Gehäuse der Vorrichtung so ausgebildet, dass die Klebeverbindung, beispielsweise die Klebestreifen oder Pflaster, die ringförmige Bandfixierung oder auch die Unterdruckflächen verlässlich und sicher mit der Vorrichtung bzw. dem Gehäuse der Vorrichtung verbunden werden können.

Daneben hat es sich insbesondere bei technischen Anwendungen der erfindungsgemäßen Vorrichtung bewährt, Fixierelemente bzw. Aufnahmen für solche Fixierelemente vorzusehen, wie beispielsweise Schrauben oder Nägel, die in den technischen Körper, der mit Hilfe des invasiven Instrumentes zu untersuchen ist, eingebracht und fixiert werden können.

Bei der Verwendung der erfindungsgemäßen Vorrichtung im medizinischen oder tiermedizinischen Bereich hat es sich als sehr vorteilhaft erwiesen, die Vorrichtung zumindest teilweise, insbesondere vollständig in einen Bissschutz, der im Bereich der Öffnung, durch die das invasive Instrument eingebracht wird, angeordnet ist, zu integrieren oder mit diesem zu verbinden. Durch diese Verbindung zwischen Bissschutz, der ein Verklemmen und dadurch eine Schädigung des Gewebes im Bereich der Öffnung beim Ein- und Ausführen des invasiven Instrumentes verhindert, ist eine sehr kompakte, einfach zu handhabende und verlässliche Struktur geschaffen, die eine verlässliche Information zur Eindringtiefe und zur rotatorischen Orientierung eines invasiven Instrumentes gewährleistet.

Im Folgenden wird die Erfindung anhand verschiedener Beispiele in den Figuren beschrieben. Die Erfindung ist nicht auf diese Beispiele beschränkt.

Es zeigen:
- Fig. 1: ein beispielhaftes, schematisches, erfindungsgemäßes System mit erfindungsgemäßer Vorrichtung und invasivem Instrument,
- Fig. 2: einen schematischen Ausschnitt einer Anordnung aus der erfindungsgemäßen Vorrichtung und invasivem Instrument,
- Fig. 3: einen beispielhaften Ausschnitt eines längenselektiven und rotationsselektiven Musters,
- Fig. 4: eine Tabelle eines beispielhaften binär codierten Musters, das längen- und rotationsselektiv ist,
- Fig. 5: eine schematische Querschnittsdarstellung einer beispielhaften erfindungsgemäßen Vorrichtung und
- Fig. 6: eine schematische Querschnittsdarstellung eines weiteren Beispiels einer erfindungsgemäßen Vorrichtung.

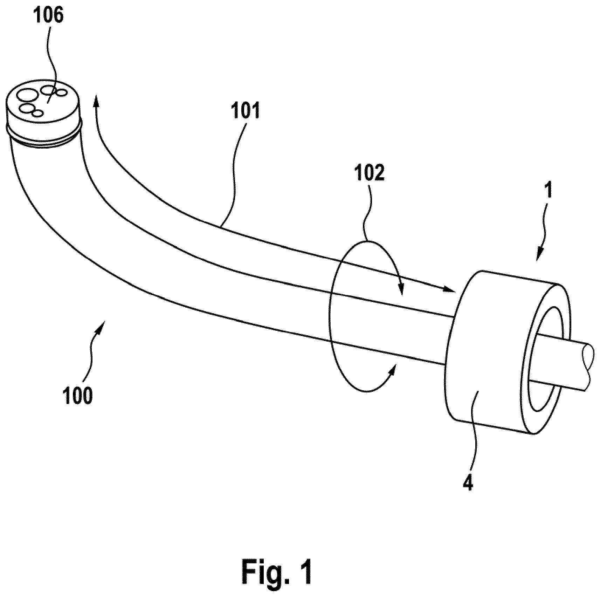

Das in Fig. 1 dargestellte System zur automatischen Erfassung der Eindringtiefe 101 und der rotatorischen Orientierung 102 eines invasiven Instrumentes 100 zeigt eine Vorrichtung 1, die das invasive Instrument 100 ringförmig umschließt. Die Vorrichtung 1 zur automatischen Erfassung der Eindringtiefe 101 und der rotatorischen Orientierung 102 weist ein ringförmiges Gehäuse 4 auf, in dem eine in Fig. 1 nicht dargestellte Auswerteeinheit 3 und ein ringförmiger Sensor 2 integriert angeordnet sind.

Der ringförmige Sensor 2 erfasst optisch die Oberfläche 103 des invasiven Instrumentes 100 und die darauf angeordneten Muster 104. Erfindungsgemäß ist auf der Oberfläche 103 des Instrumentes 100 ein längenselektives und rotationsselektives Muster 104 angeordnet, das von der Vorrichtung 1 erfasst und ausgewertet wird. Da die Vorrichtung 1 mit dem Gehäuse 4 im direkten Umfeld der Öffnung beispielsweise einer minimalinvasiven Operationsöffnung eines menschlichen Körpers angebracht ist, durch die das invasive Instrument 100, das hier als flexibles Endoskop ausgebildet ist, eingeführt wird, kann mithilfe des längenselektiven und rotationsselektiven Musters 104 im Innenbereich der Vorrichtung 1 der Abstand von der erfindungsgemäßen Vorrichtung 1 zum distalen Ende 106 des invasiven Instrumentes 100 erfasst werden. Hierzu sind diese Informationen in dem längen- und rotationsselektiven Muster im Erfassungsbereich der erfindungsgemäßen Vorrichtung codiert enthalten. Damit wird es auf direkte, einfache und sichere Weise möglich, eine Information zur Eindringtiefe 101 des medizinischen invasiven Instrumentes 100 sowie eine Information zur rotatorischen Orientierung 102 des Instrumentes 100 relativ zu der Vorrichtung 1 zu gewinnen. Diese Informationen sind für die sichere Durchführung einer Operation sehr hilfreich, da das durch das flexible Endoskop erzeugte Bild eine bessere Orientierung im Körper des Patienten ermöglicht.

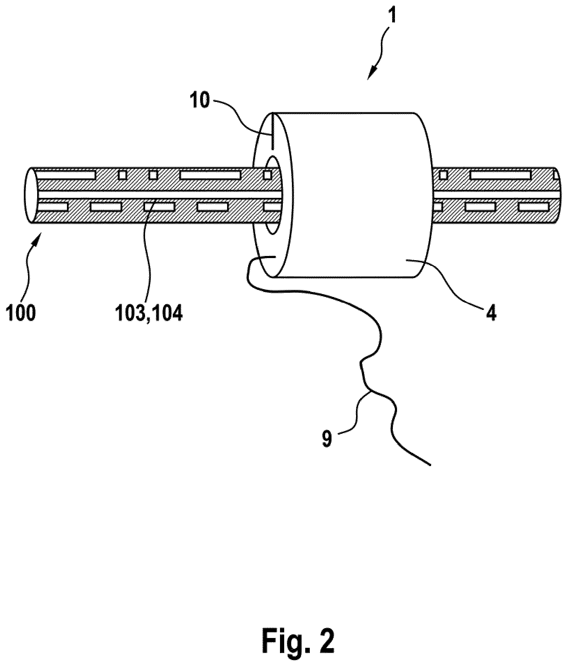

In Fig. 2 ist ein detaillierter Ausschnitt einer anderen beispielhaften erfindungsgemäßen Vorrichtung 1 dargestellt. Die Vorrichtung 1 zeigt ein hohlzylinderförmiges Gehäuse 4, durch dessen inneren Hohlraum ein invasives, starres Boreskop 100 geführt und integriert ist. Auf der Oberfläche 103 des Boreskops 100 ist ein selektives Muster 104 aufgebracht, das sich über die ganze Oberfläche 103 des invasiven Instrumentes 100 erstreckt. Die Codierung des gesamten Musters erfolgt dabei in einer Weise, dass entlang des Umfangs der Oberfläche, also im Bereich eines Querschnittes durch das Boreskop 100, eine Folge von schwarzen und weißen Bereichen angeordnet ist, die eine binäre Codierung aus einer Mehrzahl an einzelnen Bits bilden, die durch einzelne Bereiche repräsentiert sind.

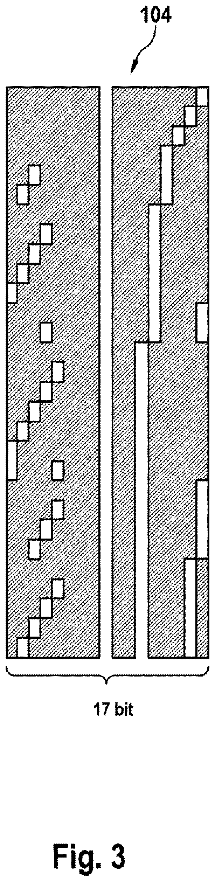

In Fig. 3 ist eine flächige Darstellung das Muster 104 wiedergegeben. Es zeigt 17 Bit Breite und ist als binärer Code aus schwarzen und weißen, nebeneinander angeordneten Bereichen dargestellt. Dadurch können grundsätzlich 2¹⁷ unterschiedliche Informationen durch die unterschiedlichen möglichen Anordnungen repräsentiert werden. Erfindungsgemäß werden aber nicht alle möglichen Codierungen verwendet, sondern nur solche, die zueinander mindestens eine Hamming-Distanz von 1 aufweisen. Erfindungsgemäß enthält jedes einzelne Codemuster wenigstens einen Übergang von weiß zu schwarz und umgekehrt von schwarz zu weiß von einem zum benachbarten Bereich. Zusätzlich werden nur solche Codemuster verwendet, die selektiv gegen zyklisches Vertauschen ausgebildet sind. Das bedeutet, dass unabhängig von der Position des Beginns des Auslesens entlang des Umfangs um das invasive Instrument 100 die Anordnung der Bereiche eindeutig ist und nicht allein durch den unterschiedlichen Aufsatzpunkt des Auslesens eine Verwechslung stattfinden kann. Dies führt dazu, dass von den 2¹⁷ theoretisch möglichen unterschiedlichen Codes nur ein kleiner Teil tatsächlich Verwendung findet, um eine eindeutige rotationsselektive Orientierungsinformation zu erhalten. In Fig. 3 sind solche Beispiele für eine solche eindeutige Codierung graphisch dargestellt. Damit ist es möglich, einerseits die Länge, also den Abstand zum distalen Ende 106 des Boreskops 100 mithilfe der erfindungsgemäßen Vorrichtung zu ermitteln und zum anderen zugleich die relative rotatorische Orientierung 102 zu ermitteln.

Durch die Verwendung eines solchen 17-Bit-langen Binärmusters mit einer Hamming-Distanz von 1 können 7.710 detektierbare Anordnungen codiert werden, die bei einer Länge eines Boreskops 100 von 7,7 m eine Längenauflösung und damit eine Auflösung der Eindringtiefe 101 von 1 mm ermöglicht. Bezüglich der Winkeltoleranz ergibt sich bei einem 17-Bit-langen Code eine Auflösung von 360°:17, also 21,1°. Es lassen sich somit mit diesem Muster 17 Orientierungsstufen mit einem Abstand von 21,1° differenziert erfassen und dem Benutzer zur Verfügung stellen.

Im Inneren der erfindungsgemäßen Vorrichtung 1 bzw. des ringförmigen Gehäuses 4 ist ein ringförmiger Sensor 2 enthalten, der geeignet und dafür ausgebildet ist, den Umfang des im inneren Hohlraum des Gehäuses 4 angeordneten Boreskops 100 zu erfassen und das auf dessen Oberfläche 103 angeordnete Muster 104 zu erfassen und mithilfe der im Gehäuse 4 angeordneten Auswerteeinheit 3 auf die Eindringtiefeninformation und die Information zur rotatorischen Orientierung 102 auszuwerten.

Die erfindungsgemäße Vorrichtung 1 wird dabei direkt auf die Körperöffnung des Rohres, in das das Boreskop 100 eingeführt wird, aufgebracht und mit diesem lösbar verbunden. Dadurch ist sichergestellt, dass die erfasste Längeninformation der Eindringtiefe 101 bzw. die erfasste rotatorische Orientierung 102 der tatsächlichen Orientierung des Boreskops 100 in der Öffnung entspricht.

Zusätzlich ist auf dem Gehäuse 4 eine Markierung 10 angeordnet, die dem Benutzer die Möglichkeit gibt, einen Bezugspunkt für die Orientierungsinformation, d.h. das Verdrehen relativ zu dieser Orientierungsmarkierung 10, zu vermitteln.

Die durch die Vorrichtung 1 ermittelte Information zur Eindringtiefe 101 und zur rotatorischen Orientierung 102 wird mithilfe eines elektrischen Kabels 9 an eine abgesetzte, hier nicht dargestellte Ausgabeeinheit, die ein Display darstellt, weitergegeben und durch diese Ausgabeeinheit dem Benutzer zur Verfügung gestellt. Die Eindringtiefe 101 wird dabei typischerweise als alphanumerische Information, beispielsweise 1,37 m ausgegeben, während die rotatorische Orientierungsinformation regelmäßig als grafische Darstellung in Form eines größeren oder kleineren Winkelsegments dargestellt wird. Wunschgemäß kann der Benutzer auch andere Darstellungsformen zur Eindringtiefe 101 oder zur rotatorischen Orientierung 102 wählen.

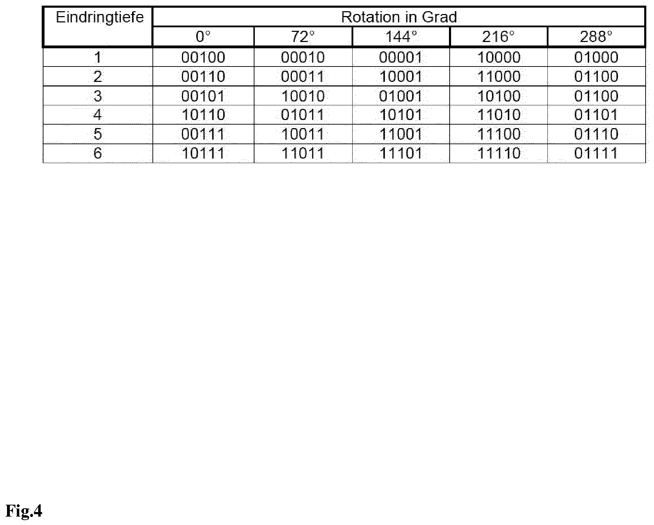

Die in Fig. 4 dargestellte Tabelle zeigt eine Codierung für ein selektives Muster, die 5 Bit lang ist. Dabei ist jedes Bit als binärer Code 0 oder 1 realisiert. Dieser 5-Bit-Binärcode kann durch Schwarz-Weiß-Markierungsbereiche entlang des Umfangs um den Schaft des invasiven Instrumentes herum aufgebracht werden und der erfinderischen Vorrichtung zur automatisierten Erfassung zugeführt werden. Zwar sind theoretisch 2⁵, also 32 unterschiedliche Codes, also Anordnungen der Bereiche möglich, jedoch hinsichtlich der Eindeutigkeit der Anordnungen auch bei einer Rotation, d.h. gegenüber zyklischem Vertauschen und insbesondere hinsichtlich einer vorgesehenen Hamming-Distanz von 1 oder größer werden eine Vielzahl der 32 möglichen Codes nicht verwendet, sondern nur sechs dieser Codes. Diese sind in der Tabelle der Fig. 4 dargestellt. Jedem der sechs Codes ist eine Eindringtiefe 101 zugeordnet. Mithilfe dieses in Fig. 4 beispielhaft dargestellten selektiven Codesystems, das als Schwarz-Weiß-Muster auf der Oberfläche 103 des invasiven Instrumentes 100 aufgebracht werden kann, können somit sechs unterschiedliche Eindringtiefen des invasiven Instrumentes unterschieden werden.

Darüber hinaus ist auch eine Information oder Aussage zu der rotatorischen Orientierung 102 möglich. Wird das invasive Instrument 100 rotatorisch verdreht, so verändert sich der relative Aufsatzpunkt für das Auslesen des Musters durch den ringförmigen Sensor 2 entlang des Umfangs auf der Oberfläche des invasiven Instrumentes 100. Abhängig von der rotatorischen Orientierung 102, d.h. dem Maß der Verdrehung, verändert sich der Startpunkt des Auslesens des Codes. Durch die vorgenannte spezifische Wahl der Codierung ist es möglich, trotz eines zyklischen Vertauschens der Reihenfolge der Codes die eindeutige Eindringtiefe 101 zu bestimmen und zusätzlich aufgrund des Maßes der zyklischen Verschiebung ein Maß für die rotatorische Verschiebung und damit die entsprechende Orientierung zu bestimmen. In diesem Fall gibt es fünf verschiedene zyklische Verschiebungsstufen, da hier ein 5-Bit-Binärcode verwendet wurde. Damit können Rotationsstufen von 0°, von 72°, von 144°, von 216° und von 288° unterschieden werden.

Liest die erfindungsgemäße Vorrichtung 1 mit dem ringförmig angeordneten Sensor 2 und der Auswerteeinheit die Bitfolge 01001 aus, so kann anhand der in Fig. 4 dargestellten Tabelle ein Rückschluss auf die Eindringtiefe 101 und das Maß der rotatorischen Orientierung 102 abgeleitet werden. Im vorliegenden Fall würde der Code 01001 eine Eindringtiefe 101 der Stufe 3 und eine Rotation um 144° bedeuten.

Durch die erfindungsgemäße Wahl der spezifischen Anordnung der Bereiche gelingt es sehr verlässlich und sicher, eine längenspezifische aber auch rotationsspezifische Information zu gewinnen und diese dem Benutzer zur Verfügung zu stellen. Durch die besondere Wahl dieser Codierungen können auch mögliche Übertragungsfehler zumindest in einem gewissen Umfang erkannt und im Rahmen des Auswerteprozesses eliminiert werden.

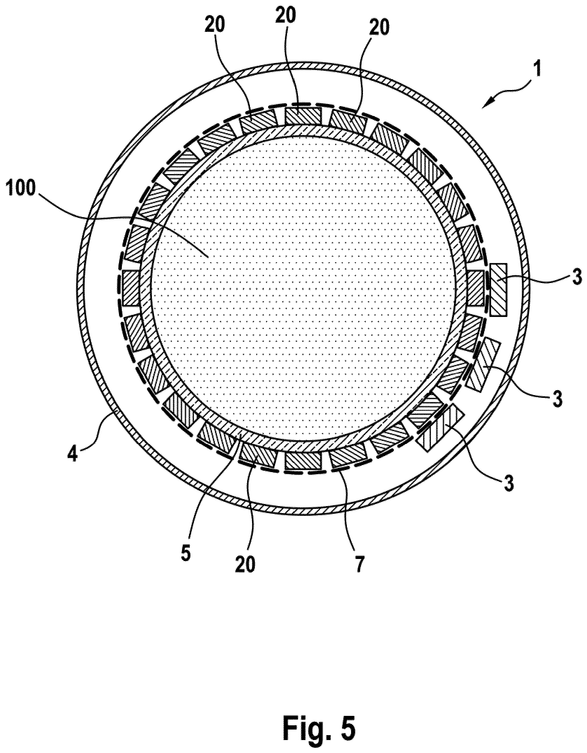

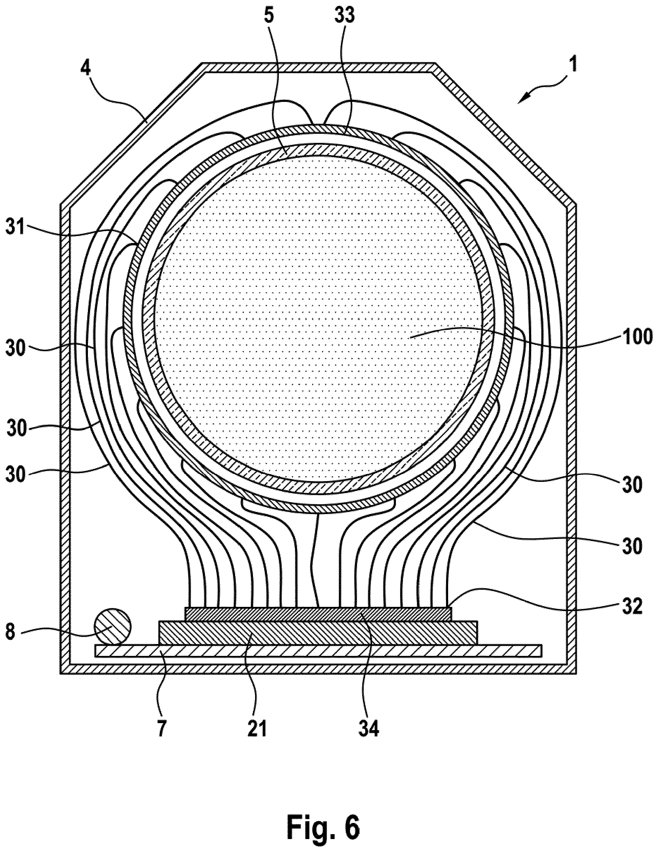

Der Aufbau der erfindungsgemäßen Vorrichtung 1 mit einem hohlzylinderförmigen Gehäuse 4, das ringförmig den Innenraum umschließt, in den das zu untersuchende invasive Instrument 100 mit den auf seiner Oberfläche angebrachten Mustern im Anwendungsfall eingebracht wird, ist in Fig. 5 bzw. Fig. 6 in Form eines Querschnittes als zwei Varianten dargestellt.

Das Gehäuse 4 umschließt ringförmig das invasive Instrument 100, wobei auf der Innenseite des Gehäuses 4, auf der dem invasiven Instrument 100 zugeordneten Seite ein optisch transparentes Innengehäuse 5 als Teil des Gehäuses angeordnet ist. Das Gehäuse 4 und das Innengehäuse 5 umschließen die erfindungsgemäße Vorrichtung 1 so dicht ab, dass die erfindungsgemäße Vorrichtung 1 effizient sterilisiert werden kann und für einen Einsatz in einem Operationssaal bei einer minimalinvasiven Operation verwendet werden kann.

Im Inneren des Gehäuses 4 im Bereich des transparenten Innengehäuses 5 ist eine Vielzahl von einzelnen Sensorelementen 20 angeordnet, die als Reflexionslichtschranken ausgebildet sind. Die Anordnung der Reflexionslichtschranken 20 umschließt ringförmig das ringförmig geschlossene, transparente Innengehäuse 5, so dass die Reflexionslichtschranken 20 in der Lage sind, die durch das transparente Innengehäuse 5 sichtbaren Muster 104 auf der Oberfläche 103 des invasiven Instrumentes 100 sicher zu erfassen. Dabei ist die Anzahl der verwendeten Reflexionslichtschranken 20 doppelt so hoch gewählt wie die Anzahl der Bits, die zur Codierung der Informationen mithilfe der Muster verwendet werden. Gemäß dem Nyquist - Shannon - Theorem kann damit eindeutig das Muster bestimmt werden. Wird z.B. ein 10-Bit langes Muster verwendet, so wird die doppelte Anzahl an Reflexionslichtschranken, also 20 Stück, im Kreis um das transparente Innengehäuse 5 angeordnet. Die durch die Reflexionslichtschranken 20 gebildeten Sensorsignale werden an eine ringförmig ausgebildete Leiterplatte 7 abgegeben, die diese Signale an drei im Gehäuse angeordnete Auswerteeinheiten 3 weiterleiten. Dabei umschließt die ringförmig ausgebildete Leiterplatte 7 die zu einem Ring angeordneten Reflexionslichtschranken 20 und gewährleistet die mechanische Stabilität dieser Anordnung, da diese auf der ringförmigen Leiterplatte 7 nicht nur elektrisch, sondern auch mechanisch gekoppelt sind. Durch diese mechanisch steife Kopplung ist eine dauerhafte, sichere und verlässliche Ausrichtung der Reflexionslichtschranken in Richtung des Innenraums des hohlzylinderförmigen Gehäuses 4 durch den transparenten Teil des Innengehäuses 5 gewährleistet.

Durch die gleichmäßig verteilte Anordnung der Reflexionslichtschranken 20 ist eine vollständige und gleichmäßige Erfassung des Innenraums gewährleistet, so dass das Erfassungsergebnis der erfindungsgemäßen Vorrichtung von hoher Qualität ist.

Während die Reflexionslichtschranken 20 auf der Innenseite der ringförmigen Leiterplatte 7 angeordnet sind, sind die Auswerteeinheiten 3 auf der außenliegenden Seite der ringförmigen Leiterplatte angeordnet, wodurch eine kompakte Anordnung der Leiterplatte mit verbundenen elektronischen Komponenten bei effizienter Erfassung des gewünschten Erfassungsbereiches gegeben ist. Diese Anordnung lässt sich dadurch in ein kompaktes ringförmiges Gehäuse 4, das die Gestalt eines Hohlzylinders aufweist, integrieren, ohne dass das Gehäuse sperrig und schwierig zu handhaben ist. Dadurch ist eine gute Handhabbarkeit auch im schwierigen Operationsumfeld gegeben.

Eine alternative Ausbildung der erfindungsgemäßen Vorrichtung 1 ist in Fig. 6 in Form einer Darstellung eines schematischen Querschnitts dargestellt. In dem Gehäuse 4, das einen Innenraum mit dem invasiven Instrument 100 ringförmig umschließt, ist ein Sensor angeordnet, der im Gegensatz zu der Vorrichtung 1 der Fig. 5 einen linearen Array 21 aus CMOS-Sensoren zeigt. Dieser lineare Array 21 ist auf einer planen Leiterplatte 7 angeordnet, die eine Kopplungsstelle 8 zur Ankopplung einer Ausgabeeinheit aufweist. Über diese Kopplungsstelle 8 werden die erfassten und ausgewerteten Informationen an die Ausgabeeinheit übertragen, die diese Informationen zur Eindringtiefe 101 und zur rotatorischen Orientierung 102 des invasiven Instrumentes 100 einem Benutzer zur Verfügung stellen kann.

Entsprechend der Vorrichtung 1 aus Fig. 5 zeigt auch dieses Gehäuse 4 ein transparentes Innengehäuse 5, durch das die Oberfläche 103 des eingebrachten invasiven Instrumentes 100 mittels des in dem Gehäuse 4 integrierten Sensors erfasst wird. Der Sensor besteht dabei zum einen aus dem linearen Array 21 und einer Vielzahl von Lichtwellenleitern 30, deren erste Enden 31 ringförmig in einem Halter 33 um das optisch transparente Innengehäuse 5 angeordnet sind und einem zweiten Halter 34 für die anderen, zweiten Enden 32 der Lichtwellenleiter 30. Die Lichtwellenleiter 30 übertragen das durch ihr erstes Ende 31 aufgenommene Licht von der Oberfläche 103 des invasiven Instrumentes 100, welches nach Durchtritt des optisch transparenten Innengehäuses 5 über das erste Ende 31 in den Lichtwellenleiter 30 zu dem anderen, zweiten Ende 32 eintritt. Dort tritt das Licht aus und wird auf das lineare Array 21 aus CMOS-Sensorelementen eingekoppelt. Die Reihenfolge der ersten Enden 31 entlang des Umfangs des ringförmigen Halters 33 und damit entlang des Umfangs des optisch transparenten Innengehäuses 5 bzw. des Umfangs des invasiven Instrumentes 100 entspricht der Reihenfolge entlang des linearen Arrays 21 an CMOS-Sensoren. Dadurch wird auf einfache und verlässliche Weise gewährleistet, dass das auf dem Umfang der Oberfläche des invasiven Instrumentes 100 angebrachte Muster 104 aus hellen und dunklen Bereichen verlässlich auf die CMOS-Sensorelemente des Arrays 21 übertragen wird und dort in ein korrektes lineares binäres Binärcodewort umgesetzt wird. Dieses wird durch die hier nicht dargestellte Auswerteeinheit, die ebenso auf der Leiterplatte 7 angeordnet ist, ausgewertet und als Information zur Eindringtiefe 101 bzw. zur rotatorischen Orientierung 102 über die Kopplung 8 an eine angeschlossene Ausgabeeinheit weitergeleitet. Da die Lichtwellenleiter 30 einen sehr geringen Durchmesser haben, ist es möglich, eine Vielzahl solcher Lichtwellenleiter 30 eng benachbart entlang des Umfangs des transparenten Innengehäuses 5 anzuordnen und dadurch eine sehr hohe Auflösung des Sensors 2 zu ermöglichen. Weiterhin ist die Anordnung von CMOS-Sensorelementen auf einem linearen Array 21 auch technisch einfach mit einer hohen Auflösung, d.h. einer hohen Anzahl an CMOS-Sensorelementen je Zentimeter möglich. Dies führt insgesamt zu einem sehr hochauflösenden Sensor 2 bestehend aus dem Array 21, aus CMOS-Sensorelementen, der Vielzahl von Lichtwellenleitern 30, deren ersten Enden 31 in einem Halter 33 ringförmig das transparente Innengehäuse 5 umschließen und den zweiten Enden 32, die mit einem linear oder planen Halter 34 mit dem Array 21 koppeln. Diese Ausbildung der erfindungsgemäßen Vorrichtung zeichnet sich durch eine ausgesprochen hohe Auflösung aus, die es ermöglicht, auch Muster mit einer großen Anzahl an Einzelbits, also an einzelnen flächigen Bereichen unterschiedlicher Farbe und/oder Helligkeit zu erfassen und einer Auswertung durch die Auswerteeinheit zuzuführen.

Diese erfindungsgemäßen Vorrichtungen 1 ermöglichen ein sehr sicheres, verlässliches Erfassen der Eindringtiefe 101 bzw. der rotatorischen Orientierung 102 eines eingebrachten invasiven Instrumentes.

### Bezugszeichenliste

- 1: Vorrichtung zur automatischen Erfassung der Eindringtiefe 101 und der rotatorischen Orientierung 102 eines invasiven Instrumentes
- 2: ringförmiger Sensor
- 3: Auswerteeinheit
- 4: ringförmiges Gehäuse
- 5: ringförmiges, optisch transparentes Innengehäuse
- 7: Leiterplatte
- 8: Kopplungsstelle zu einer Ausgabeeinheit
- 9: Kabel
- 10: Referenzmarkierung Orientierung
- 20: Sensorelemente
- 21: Array aus Sensorelementen
- 30: Lichtwellenleiter
- 31: erstes Ende eines Lichtwellenleiters
- 32: zweites Ende eines Lichtwellenleiters
- 33: Halter für die ersten Enden der Lichtwellenleiter
- 34: Halter für die zweiten Enden der Lichtwellenleiter
- 100: Invasives Instrument
- 101: Eindringtiefe eines invasiven Instrumentes
- 102: Rotatorische Orientierung eines invasiven Instrumentes
- 103: Oberfläche eines invasiven Instrumentes
- 104: selektives Muster
- 106: distales Ende des invasiven Instrumentes

## Patentansprüche

1. Vorrichtung (1) zur automatischen Erfassung der Eindringtiefe (101) eines invasiven Instrumentes (100) in eine Öffnung und der rotatorischen Orientierung (102) des invasiven Instrumentes (100) in einer Öffnung eines Körpers mithilfe eines auf der Oberfläche (103) des Instrumentes (100) angebrachten längenselektiven und rotationsselektiven Musters (104), wobei die Vorrichtung (1) einen Sensor (2) aufweist, wobei der Sensor (2) zur Erfassung des längenselektiven und rotationsselektiven Musters (104) ausgebildet ist, wobei der Sensor (2) zur zeitweiligen Anordnung im Bereich der Öffnung für das Einbringen des invasiven Instrumentes (100) ausgebildet ist und wobei die Vorrichtung (1) eine Auswerteeinheit (3) zur Auswertung der Eindringtiefe (101) und der rotatorischen Orientierung (102) des invasiven Instrumentes (100) anhand des erfassten selektiven Musters (104) aufweist, **dadurch gekennzeichnet, dass** der Sensor (2) zur Umschließung des Instrumentes (100) ringförmig ausgebildet ist und aus mehreren ringförmig angeordneten Sensorelementen (20), insbesondere aus CCD-Sensorelementen, CMOS- Sensorelementen und/oder Reflexlichtschranken-Sensorelementen gebildet ist.

2. Vorrichtung zur automatischen Erfassung der Eindringtiefe (101) und der rotatorischen Orientierung (102) eines invasiven Instrumentes nach Anspruch 1, **dadurch gekennzeichnet, dass** die Auswerteeinheit (3) in einem ringförmigen Gehäuse (4) mit dem ringförmigen Sensor (2) angeordnet ist.

3. Vorrichtung zur automatischen Erfassung der Eindringtiefe (101) und der rotatorischen Orientierung (102) eines invasiven Instrumentes nach Anspruch 2, **dadurch gekennzeichnet, dass** das ringförmige Gehäuse (4) so aufklappbar ausgebildet ist, dass der ringförmige Sensor (2) mit dem ringförmigen Gehäuse (4) geöffnet und geschlossen werden kann.

4. Vorrichtung zur automatischen Erfassung der Eindringtiefe (101) und der rotatorischen Orientierung (102) eines invasiven Instrumentes nach einem der Ansprüche 2 bis 3, **dadurch gekennzeichnet, dass** das ringförmige Gehäuse (4) auf der im Betriebszustand dem invasiven Instrument (100) zugewandten Seite ein optisch transparentes Innengehäuse (5) aufweist.

5. Vorrichtung zur automatischen Erfassung der Eindringtiefe (101) und der rotatorischen Orientierung (102) eines invasiven Instrumentes nach einem der Ansprüche 1 bis 4, **dadurch gekennzeichnet, dass** der ringförmige Sensor (2) mehrere Lichtwellenleiter (30) aufweist, deren erste Enden (31) ringförmig im Gehäuse (4) angeordnet sind und deren zweite Enden (32) mehreren Sensorelementen (20) insbesondere aus CCD-Sensorelementen, CMOS- Sensorelementen und/oder Reflexlichtschranken-Sensorelementen zugeordnet sind, die arrayförmig, insbesondere als lineares Array (21), angeordnet sind.

6. Vorrichtung zur automatischen Erfassung der Eindringtiefe (101) und der rotatorischen Orientierung (102) eines invasiven Instrumentes nach einem der vorstehenden Ansprüche 2 bis 5, **dadurch gekennzeichnet, dass** das ringförmige Gehäuse (4) mit dem ringförmigen Sensor (2) eine insbesondere ringförmige Lichtquelle aufweist, die den durch das ringförmige Gehäuse (4) umschlossenen Innenbereich (10) ringförmig beleuchtet.

7. Vorrichtung zur automatischen Erfassung der Eindringtiefe (101) und der rotatorischen Orientierung (102) eines invasiven Instrumentes nach einem der vorstehenden Ansprüche, **dadurch gekennzeichnet, dass** die Vorrichtung (1) mehrere parallel zueinander angeordnete ringförmige Sensoren (2) zur Erfassung des längenselektiven und rotationsselektiven Musters (104) aufweist, deren Sensorsignal wenigstens teilweise gemeinsam mithilfe der Auswerteeinheit (3) ausgewertet wird.

8. Vorrichtung zur automatischen Erfassung der Eindringtiefe (101) und der rotatorischen Orientierung (102) eines invasiven Instrumentes nach einem der vorstehenden Ansprüche, **dadurch gekennzeichnet, dass** das Gehäuse (4) eine ringförmige oder teilringförmige Leiterplatte (7) aufweist, die die Auswerteeinheit (3), die Ansteuerung des oder der Sensoren (2) und/oder der Lichtquelle oder Teile davon enthält.

9. Vorrichtung zur automatischen Erfassung der Eindringtiefe (101) und der rotatorischen Orientierung (102) eines invasiven Instrumentes nach einem der vorstehenden Ansprüche, **dadurch gekennzeichnet, dass** die Vorrichtung (1) mit einer Ausgabeeinheit verbunden ist, die geeignet ist, von der Auswerteeinheit (3) ausgewertete Eindringtiefen (101) und rotatorische Orientierungen (102) eines invasiven Instrumentes (100), insbesondere optisch einem Benutzer der Vorrichtung (1) zur Verfügung zu stellen.

10. Vorrichtung zur automatischen Erfassung der Eindringtiefe (101) und der rotatorischen Orientierung (102) eines invasiven Instrumentes nach einem der vorstehenden Ansprüche 2 bis 9, **dadurch gekennzeichnet, dass** das ringförmige Gehäuse (4) zur lösbaren Befestigung an dem Körper ausgebildet ist.

11. Vorrichtung zur automatischen Erfassung der Eindringtiefe (101) und der rotatorischen Orientierung (102) eines invasiven Instrumentes nach einem der vorstehenden Ansprüche, **dadurch gekennzeichnet, dass** die Vorrichtung (1) zumindest teilweise in einen Bissschutz integriert oder mit diesem verbunden ist.

12. System zur automatischen Erfassung der Eindringtiefe (101) und der rotatorischen Orientierung (102) eines invasiven Instrumentes (100) mit einer Vorrichtung (1) nach einem der vorstehenden Ansprüche und einem invasiven Instrument (100), wobei auf dem Umfang des Instrumentes (100) auf der Oberfläche (103) ein längenselektives und rotationsselektives Muster (104) angeordnet ist, das aus Bereichen unterschiedlicher Farbe und Helligkeit besteht, deren Anordnung sich bei unterschiedlichen Längen und bei unterschiedlichen rotatorischen Orientierungen selektiv unterscheidet, wobei das Muster (104) ein binäres Muster (104) aus Bereichen von zwei unterschiedlichen Farben und/oder Helligkeiten darstellt und die verschiedenen Anordnungen der Bereiche des Musters (104) entlang des Instruments eine Hamming-Distanz von wenigstens 1 aufweisen.

13. System zur automatischen Erfassung der Eindringtiefe (101) und der rotatorischen Orientierung (102) eines invasiven Instrumentes nach Anspruch 12, **dadurch gekennzeichnet, dass** jede Anordnung der Bereiche des Musters (104) auch bei zyklischem Vertauschen der Bereiche eindeutig ausgebildet ist.

## Claims

1. Device (1) for automatically registering the penetration depth (101) of an invasive instrument (100) into an opening and the rotational orientation (102) of the invasive instrument (100) in an opening of a body with the aid of a length-selective and rotation-selective pattern (104) applied to the surface (103) of the instrument (100), wherein the device (1) has a sensor (2), wherein the sensor (2) is embodied for registering the length-selective and rotation-selective pattern (104), wherein the sensor (2) is embodied for the temporary arrangement in the region of the opening for the insertion of the invasive instrument (100), and wherein the device (1) has an evaluation unit (3) for evaluating the penetration depth (101) and the rotational orientation (102) of the invasive instrument (100) on the basis of the registered selective pattern (104), **characterized in that** the sensor (2) is embodied to surround the instrument (100) in a ring-shaped manner and is formed from a plurality of sensor elements (20) arranged in a ring-shaped manner, in particular of CCD sensor elements, CMOS sensor elements and/or reflection-light photoelectric sensor elements.

2. Device for automatically registering the penetration depth (101) and the rotational orientation (102) of an invasive instrument according to Claim 1, **characterized in that** the evaluation unit (3) is arranged in a ring-shaped housing (4) with the ring-shaped sensor (2).

3. Device for automatically registering the penetration depth (101) and the rotational orientation (102) of an invasive instrument according to Claim 2, **characterized in that** the ring-shaped housing (4) is embodied in such a hinged manner that the ring-shaped sensor (2) can be opened and closed with the ring-shaped housing (4).

4. Device for automatically registering the penetration depth (101) and the rotational orientation (102) of an invasive instrument according to one of Claims 2 and 3, **characterized in that** the ring-shaped housing (4) has an optically transparent inner housing (5) on the side facing the invasive instrument (100) in the operational state.

5. Device for automatically registering the penetration depth (101) and the rotational orientation (102) of an invasive instrument according to one of Claims 1 to 4, **characterized in that** the ring-shaped sensor (2) has a plurality of optical waveguides (30), the first ends (31) of which are arranged in the housing (4) in a ring-shaped manner and the second ends (32) of which are assigned to a plurality of sensor elements (20), in particular of CCD sensor elements, CMOS sensor elements and/or reflection-light photoelectric sensor elements, which are arranged in an array-shaped manner, in particular as a linear array (21).

6. Device for automatically registering the penetration depth (101) and the rotational orientation (102) of an invasive instrument according to one of the preceding Claims 2 to 5, **characterized in that** the ring-shaped housing (4) with the ring-shaped sensor (2) has an - in particular ring-shaped - light source, which illuminates the inner region (10) enclosed by the ring-shaped housing (4) in a ring-shaped manner.

7. Device for automatically registering the penetration depth (101) and the rotational orientation (102) of an invasive instrument according to one of the preceding claims, **characterized in that** the device (1) has a plurality of ring-shaped sensors (2) arranged parallel to one another for registering the length-selective and rotation-selective pattern (104), the sensor signal of which sensors is at least partly evaluated together with the aid of the evaluation unit (3).

8. Device for automatically registering the penetration depth (101) and the rotational orientation (102) of an invasive instrument according to one of the preceding claims, **characterized in that** the housing (4) has a ring-shaped or partial ring-shaped circuit board (7), which contains the evaluation unit (3), the actuation of the sensor or sensors (2) and/or of the light source or parts thereof.

9. Device for automatically registering the penetration depth (101) and the rotational orientation (102) of an invasive instrument according to one of the preceding claims, **characterized in that** the device (1) is connected to an output unit suitable for providing penetration depths (101) and rotational orientations (102) of an invasive instrument (100) as evaluated by the evaluation unit (3) to a user of the device (1), in particular optically.

10. Device for automatically registering the penetration depth (101) and the rotational orientation (102) of an invasive instrument according to one of the preceding Claims 2 to 9, **characterized in that** the ring-shaped housing (4) is embodied for detachable fastening to the body.

11. Device for automatically registering the penetration depth (101) and the rotational orientation (102) of an invasive instrument according to one of the preceding claims, **characterized in that** the device (1) is at least partly integrated into a nip protection or connected thereto.

12. System for automatically registering the penetration depth (101) and the rotational orientation (102) of an invasive instrument (100) with a device (1) according to one of the preceding claims and an invasive instrument (100), wherein a length-selective and rotation-selective pattern (104) is arranged on the circumference of the instrument (100) on the surface (103) thereof, which pattern consists of regions with different color and brightness, the arrangement of which selectively differs in the case of different lengths and different rotational orientations, wherein the pattern (104) constitutes a binary pattern (104) of regions of two different colors and/or brightness levels and the different arrangements of the regions of the pattern (104) along the instrument have a Hamming distance of at least 1.

13. System for automatically registering the penetration depth (101) and the rotational orientation (102) of an invasive instrument according to Claim 12, **characterized in that** each arrangement of the regions of the pattern (104) also has a unique embodiment in the case of cyclical transposition of the regions.

## Revendications

1. Dispositif (1) de détection automatique de la profondeur de pénétration (101) d'un instrument invasif (100) dans une ouverture et de l'orientation en rotation (102) de l'instrument invasif (100) dans une ouverture d'un corps à l'aide de motifs (104), sélectifs en termes de longueur et de rotation, qui sont montés sur une surface (103) de l'instrument (100), le dispositif (1) comprenant un capteur (2), le capteur (2) étant conçu pour détecter le motif (104) sélectif en termes de longueur et de rotation, le capteur (2) étant conçu pour être disposé temporairement dans la région de l'ouverture destinée à l'introduction de l'instrument invasif (100) et le dispositif (1) comportant une unité d'évaluation (3) destinée à évaluer la profondeur de pénétration (101) et l'orientation en rotation (102) de l'instrument invasif (100) sur la base du motif sélectif (104) détecté, **caractérisé en ce que** le capteur (2) a une forme annulaire de manière à enfermer l'instrument (100) et est formé d'une pluralité d'éléments de capteur (20) disposés annulairement, en particulier d'éléments de capteur CCD, d'éléments de capteur CMOS et/ou d'éléments de capteur à barrière lumineuse à réflexion.

2. Dispositif de détection automatique de la profondeur de pénétration (101) et de l'orientation en rotation (102) d'un instrument invasif selon la revendication 1, **caractérisé en ce que** l'unité d'évaluation (3) est disposée conjointement avec le capteur annulaire (2) dans un boîtier annulaire (4).

3. Dispositif de détection automatique de la profondeur de pénétration (101) et de l'orientation en rotation (102) d'un instrument invasif selon la revendication 2, **caractérisé en ce que** le boîtier annulaire (4) est conçu pour être pliable de sorte que le capteur annulaire (2) peut être ouvert et fermé avec le boîtier annulaire (4).

4. Dispositif de détection automatique de la profondeur de pénétration (101) et de l'orientation en rotation (102) d'un instrument invasif selon l'une des revendications 2 à 3, **caractérisé en ce que** le boîtier annulaire (4) comporte un boîtier intérieur (5) optiquement transparent sur le côté faisant face à l'instrument invasif (100) à l'état de fonctionnement.

5. Dispositif de détection automatique de la profondeur de pénétration (101) et de l'orientation en rotation (102) d'un instrument invasif selon l'une des revendications 1 à 4, **caractérisé en ce que** le capteur annulaire (2) comporte une pluralité de fibres optiques (30), dont les premières extrémités (31) sont disposées annulairement dans le boîtier (4) et dont les deuxièmes extrémités (32) sont associées à une pluralité d'éléments de capteur (20), notamment formés d'éléments de capteur CCD, d'éléments de capteur CMOS et/ou d'éléments de capteur à barrière lumineuse à réflexion, qui sont disposés en matrice, notamment en matrice linéaire (21).

6. Dispositif de détection automatique de la profondeur de pénétration (101) et de l'orientation en rotation (102) d'un instrument invasif selon l'une des revendications 2 à 5 précédentes, **caractérisé en ce que** le boîtier annulaire (4) pourvu du capteur annulaire (2) comporte une source de lumière notamment annulaire qui éclaire annulairement la région intérieure (10) enfermée par le boîtier annulaire (4).

7. Dispositif de détection automatique de la profondeur de pénétration (101) et de l'orientation en rotation (102) d'un instrument invasif selon l'une des revendications précédentes, **caractérisé en ce que** le dispositif (1) comporte une pluralité de capteurs annulaires (2), parallèles entre eux, qui sont destinés à détecter le motif (104) sélectif en longueur et en rotation et dont le signal de capteur est évalué de manière au moins partiellement commune, à l'aide de l'unité d'évaluation (3).

8. Dispositif de détection automatique de la profondeur de pénétration (101) et de l'orientation en rotation (102) d'un instrument invasif selon l'une des revendications précédentes, **caractérisé en ce que** le boîtier (4) comporte une carte de circuit imprimé (7) annulaire ou partiellement annulaire qui contient l'unité d'évaluation (3), la commande de l'au moins un capteur (2) et/ou de la source de lumière ou des parties de ceux-ci.

9. Dispositif de détection automatique de la profondeur de pénétration (101) et de l'orientation en rotation (102) d'un instrument invasif selon l'une des revendications précédentes, **caractérisé en ce que** le dispositif (1) est relié à une unité de sortie adaptée pour fournir des profondeurs de pénétration (101) et des orientations de rotation (102) d'un instrument invasif (100), lesquelles ont été évaluées par l'unité d'évaluation (3), en particulier visuellement à un utilisateur du dispositif (1).

10. Dispositif de détection automatique de la profondeur de pénétration (101) et de l'orientation en rotation (102) d'un instrument invasif selon l'une des revendications 2 à 9 précédentes, **caractérisé en ce que** le boîtier annulaire (4) est conçu pour être fixé au corps de manière amovible.

11. Dispositif de détection automatique de la profondeur de pénétration (101) et de l'orientation en rotation (102) d'un instrument invasif selon l'une des revendications précédentes, **caractérisé en ce que** le dispositif (1) est au moins partiellement intégré dans une protection anti-morsure ou relié à celui-ci.

12. Système de détection automatique de la profondeur de pénétration (101) et de l'orientation en rotation (102) d'un instrument invasif (100), ledit système comprenant un dispositif (1) selon l'une des revendications précédentes et un instrument invasif (100), un motif (104) sélectif en longueur et en rotation étant disposé sur la surface (103) située sur la périphérie de l'instrument (100) et comprenant des zones de couleur et de luminosité différentes dont la disposition diffère sélectivement avec différentes longueurs et différentes orientations en rotation, le motif (104) représentant un motif binaire (104) formé de régions de deux couleurs et/ou luminosités différentes et les différentes dispositions des régions du motif (104) le long de l'instrument ayant une distance de Hamming d'au moins 1.

13. Système de détection automatique de la profondeur de pénétration (101) et de l'orientation en rotation (102) d'un instrument invasif selon la revendication 12, **caractérisé en ce que** chaque disposition des régions du motif (104) est formée de manière univoque même lorsque les zones sont permutées de manière cyclique.
